# EUROPEAN PATENT SPECIFICATION

(11) **EP 1 565 195 B1**
(45) Date of publication and mention of the grant of the patent: **23.07.2008**
(21) Application number: 03759049.4
(22) Date of filing: 31.10.2003
(51) Int. Cl.: A61K 33/30, A61P 35/00, A61P 31/12

(54) **ANTICANCER OR ANTIVIRAL COMPOSITION**
ANTIKREBS- UND ANTIVIRALE ZUSAMMENSETZUNG
COMPOSITION ANTICANCEREUSE OU ANTIVIRALE

(30) Priority: 31.10.2002 KR 2002067291; 31.10.2002 KR 2002067293; 20.12.2002 KR 2002081951; 17.10.2003 KR 2003072732
(43) Date of publication of application: 24.08.2005
(73) Proprietor: Park, Lae-Ok, Seoul 137-814 (KR)
(72) Inventor: Park, Lae-Ok, Seoul 137-814 (KR)
(74) Representative: Eisenführ, Speiser & Partner
(86) International application number: PCT/KR2003/002324
(87) International publication number: WO 2004/039379

(56) References cited:
- WO-A-01/58414
- WO-A-91/09524
- WO-A-03/077899
- WO-A1-95/10534
- JP-A- 2000 212 088
- KR-A- 20030 021 857
- KR-A- 20030 075 124
- KR-B1- 930 002 831
- US-A- 5 518 714
- US-A- 5 719 134

## Description

### TECHNICAL FIELD

The present invention, in general, relates to an anticancer or antiviral composition, and more particularly, to an anticancer or antiviral composition comprising therapeutically effective amounts of citric acid and/or zinc and L-arginine, along with a pharmaceutically acceptable carrier.

### PRIOR ART

With development of chemotherapy, survival and recovery rates of cancer patients have improved. However, anticancer agents are problematic in terms of being highly toxic and thus severely damaging normal cells. To overcome such a side effect of anticancer agents, many recent studies have focused on developing alternative anticancer substances capable of specifically suppressing proliferation of tumor cells, leading to finding many candidates having anticancer activity. Such anticancer compounds are disclosed in International Pat. Publication Nos. WO 96/40142, WO 97/13771 and WO 95/23141. However, the newly discovered anticancer compounds, which specifically suppress proliferation of tumor cells, are chemically synthesized and thus have a potential to induce severe side effects in the body.

Such a disadvantage of the conventional anticancer agents drove many researchers to develop anticancer agents having mild side effects as well as excellent anticancer activity, and some of them have attempted to find natural substances having anticancer activity. In this regard, the present invention is related to the fact that natural human seminal fluid has an anticancer effect versus epithelial ovarian cancer.

Epithelial ovarian cancer is the most common type of ovarian cancer, and a major cause of death by malignancy in gynecological oncology. The high mortality of patients with epithelial ovarian cancer is due to insidious development of epithelial ovarian cancer, causing most patients to recognize significant symptoms at a considerably advanced stage (Ozols, R. F. Semin. Oncol., 1995, Vol.22, p61.). Despite removal of tumor by surgical operation or positive chemotherapy, only 20-30% of patients with epithelial ovarian cancer survive owing to rapid emergence of drug resistance.

Until now, risk factors and causes of epithelial ovarian cancer are still not well known. However, a large number of studies suggest that there is a relationship between frequency of ovulation and development of epithelial ovarian cancer, and such a relationship is demonstrated by the fact that incidence of epithelial ovarian cancer is increased in young and elderly women (early menarche and late menopause), unmarried women, and women without previous pregnancy (Franceshi et al. Int. J. Cancer, 1991, Vol. 49, p57; Taylor et al. Cancer, 1959, Vol. 12, p1207; Fraumeni et al. J. Natl. Cancer Inst., 1969, Vol. 42, p455; Weiss et al. J. Natl. Cancer Inst., 1977, Vol. 58, p913; Nergri et al. Int. J. Cancer, 1991, Vol. 49, p50; Stanford, J. L. Contraception 43, 1991, p543; and Franceshi et al. Int. J. Cancer, 1991, Vol. 49, p61.). Oral contraceptives are generally known to have protective effect versus development of epithelial ovarian cancer.

According to a popular theory, epithelial ovarian cancer occurs by repeated division and repair of the ovarian surface epithelium during ovulation (Fathalla, M. F. Lancet., 1971, Vol.2, p163.). During recovery of the epithelial surface, transformed epithelial cells are spontaneously mutated, tumor-suppressor genes are inactivated, and oncogenes are easily activated by carcinogens.

On the other hand, the human seminal fluid has been reported to have the following physiological functions and anticancer effects. The seminal plasma repairs immunological damage to sperm caused by cytotoxic lymphocytes after sexual intercourse (Stities et al. Nature, 1975, Vol. 253, p727; James et al. Immunol., 1985, Vol. 6, p61.), and the human seminal fluid inhibits development of humoral immunity and in vivo growth of tumor (Anderson et al. Immunol., 1985, Vol. 128, p535; and Michaelis et al. Anticancer Drugs, 2000, Vol. 11, p369.). Also, the human seminal fluid influences production of matrix metalloproteinase (MMP)-2 and MMP-9 mRNA in cervical epithelial carcinoma cells, and thus is believed to affect progression of cervical cancer upon sexual activity (Jeremias et al. Am. J. Obstet. Gynecol., 1999, Vol. 181, p591.). According to a recent report, bovine seminal ribonuclease (BS-RNase) induces apoptosis in human lymphocytes and human tumor cells in time and dose-dependent manners. BS-RNase exerts selective cytotoxicity to neuroblastoma (NB) cells resistant to chemotherapeutic drugs (Cinatl et al. Anticancer Res., 2000, Vol. 20, p853; Cinatl et al. Int. J. Oncol., 1999, Vol 15, p1001.).

In addition, Gjorgov performed ecological research on anticancer effects of seminal fluid by comparing various cases, in which relations between reduced exposure to the human seminal fluid and incidence of breast cancer were investigated. In this research, when comparing incidence risk of breast cancer in women using barrier contraceptives (condom) to that in women using non-barrier contraceptives (contraceptive drugs, intra uterine device (IUD), rhythm control, or tubal ligation), women using barrier contraceptives (condom) were found to have 5.2-fold higher incidence of breast cancer (Gjorgov et al. Folia Med., 1998, Vol. 40, p17.).

Major components of the seminal fluid having anticancer activity as described above include albumin, lactoferrin, transferrin, immunoglobulins, acid phosphatase, L-carnitine, L-arginine, L-histidine, citric acid, fructose, magnesium, zinc, prostaglandin, and glycerophosphocholine.

Based on the fact that the human seminal components have anticancer activity, the present inventors intended to develop uses of anticancer agents capable of showing excellent anticancer effect in addition to having reduced side effects.

On the other hand, the present inventors gave attention to the fact that papillomavirus causes cervical carcinomas.

Papillomavirus is known to infect epithelial cells of several tissues in animals and induce benign tumors generally called "warts" in hands, foots, the skin, the larynx, etc. To date, over 100 distinct human papillomavirus (HPV) genotypes were identified. Of them, several genotypes were reported to have specificities for infection sites and cause various diseases (Broker et al., Cold Spring Harbor Laboratory, 1989, p17.). Although most HPV infections were not treated effectively and thus caused severe pains in patients, they did not attract public attraction because of not developing fatal diseases. However, recently, specific types of HPV, especially, HPV types 16 and 18 were reported to relate to occurrence of malignant tumors in genital organs, the oral cavity, the skin, etc., and to serve as a major factor causing uterine cancer accounting for over 90% of cervical cancer, as well as HPV types 6b and 11 were revealed to cause benign tumors called "condyloma acuminata (or genital warts)" in genital organs. In addition, a large amount of evidence from epidemiological studies suggests that uterine carcinomas are caused by infection with papillomavirus, where the evidences include the findings that uterine carcinomas are mainly developed by factors spread via sexual contacts (Durst et al., Natl. Acad. Sci., 1983, Vol. 80, p3812.); and 85 to 100% of premalignant lesions such as cervical intraepithelial neoplasia (CIN) is caused by papillomavirus infection (Hansen, H., Science, 1991, Vol. 254, p1173.).

In addition, the HPV types 16 and 18 are present in 50 to 70% and 15 to 25%, respectively, of uterine cancer. However, over 25% of cancer patients infected with HPV-16 and over 50% of cancer patients infected with HPV-18 were evaluated to develop metastatic tumors (Lorincz et al., Obstetrics and Gynecology, 1992, VoL 79, p328.).

Therefore, based on the fact that uterine cancer is developed by papillomavirus, the present invention intended to prevent and treat various diseases caused by viral infections using antiviral substances present in human seminal fluid with anticancer effect, including citric acid, zinc and L-arginine.

WO 91/09524 A discloses a composition comprising 5.7% (w/w) I-arginine, 2.35% (w/w) citric acid and 0.01 % zinc for the treatment of AIDS or cancer.

US-A-5,719,134 discloses a composition comprising 1% (w/w) I-arginine, 0.8% (w/w) citric acid and 0.02% zinc for the treatment of AIDS.

WO 01/58414 A discloses a composition comprising 2.2% (w/w) I-arginine, citric acid and 0.15% zinc.

US-A-5,518,714 discloses a composition comprising 5% (w/w) I-arginine, 5% (w/w) citric acid and 5% zinc.

Based on the fact that reduced exposure to the human seminal fluid during ovulation is a pathological risk factor in epithelial ovarian cancer progression, the present inventors concluded that the human seminal fluid is able to effectively remove malignant transformed epithelial cells. In addition, the present inventors found that a composition containing the human seminal fluid with anticancer effect suppresses production of viral proteins in cervical carcinoma cells in which the cancer is caused by papillomavirus infection.

Therefore, the present invention provides uses of anticancer or antiviral compositions, as follows.

In an aspect, the present invention provides use of an anticancer composition comprising therapeutically effective amounts of citric acid, zinc and L-arginine along with a pharmaceutically acceptable carrier, according to claim 1.

In another aspect, the present invention provides uses of an antiviral composition comprising therapeutically effective amounts of citric acid, zinc and L-arginine along with a pharmaceutically acceptable carrier, according to claim 2.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a graph showing effects of an anticancer composition comprising citric acid and L-arginine as active components on viability of the human ovarian adenocarcinoma cell line SKOV-3 (derived from malignant ascites) (▲) and the human glioblastoma cell line U87 (◆), where cellular viability was analyzed by MTT assay;
FIG. 2 is a graph showing effects of an anticancer composition comprising zinc and L-arginine as active components on viability of the human ovarian adenocarcinoma cell line SKOV-3 (▲) and the human glioblastoma cell line U87 (◆), where cellular viability was analyzed by MTT assay;
FIG. 3 is a graph showing effects of an anticancer composition comprising citric acid, zinc and L-arginine as active components according to the present invention on viability of the human ovarian adenocarcinoma cell line SKOV-3 (▲), the human ovarian adenocarcinoma cell line NIH:OVCAR-3 (◆) and the human ovarian surface normal epithelial cell line NOSE (■), where cellular viability was analyzed by MTT assay;
FIG. 4 is a graph showing effects of an anticancer composition comprising citric acid, zinc and L-arginine as active components according to the present invention on viability of the human cervical carcinoma cell lines Ca Ski (▲), HeLa (◆) and C-33 A (■), where cellular viability was analyzed by MTT assay;
FIG. 5 is a result of flow cytometric analysis showing effects of an anticancer composition comprising citric acid, zinc and L-arginine as active components according to the present invention on cell cycle of the human cervical carcinoma cell line Ca Ski;
FIG. 6 is a result of flow cytometric analysis showing effects of an anticancer composition comprising citric acid, zinc and L-arginine as active components according to the present invention on cell cycle of the human cervical carcinoma cell line HeLa;
FIG. 7 is a result of flow cytometric analysis showing effects of an anticancer composition comprising citric acid, zinc and L-arginine as active components according to the present invention on cell cycle of the human cervical carcinoma cell line C-33 A;
FIG. 8 is a photograph showing a result of electrophoresis on an agarose gel of DNA extracted from the human cervical carcinoma cell lines Ca Ski, HeLa and C-33 A, which have been treated with an anticancer composition comprising citric acid, zinc and L-arginine as active components according to the present invention;
FIG. 9 is a photograph showing expression of P53 protein in the human cervical carcinoma cell lines Ca Ski, HeLa and C-33 A, which have been treated with an anticancer composition comprising citric acid, zinc and L-arginine as active components according to the present invention, by immunoblotting;
FIG. 10 is a photograph showing expression of P21 protein in the human cervical carcinoma cell lines Ca Ski, HeLa and C-33 A, which have been treated with an anticancer composition comprising citric acid, zinc and L-arginine as active components according to the present invention, by immunoblotting;
FIG. 11 is a photograph showing expression of C-Myc protein in the human cervical carcinoma cell lines Ca Ski, HeLa and C-33 A, which have been treated with an anticancer composition comprising citric acid, zinc and L-arginine as active components according to the present invention, by immunoblotting;
FIG. 12 shows DAPI-stained nuclei of the human cervical carcinoma cell lines Ca Ski, HeLa and C-33 A, which were treated with an anticancer composition comprising citric acid, zinc and L-arginine as active components according to the present invention;
FIG. 13 shows expression levels of E6 and E7 proteins of human papillomavirus (HPV) type 16 or 18 in the human cervical carcinoma cell lines Ca Ski and HeLa, which have been treated with an antiviral composition comprising citric acid, zinc and L-arginine as active components according to the present invention, by immunoblotting;
FIG. 14 shows a therapeutic effect versus condyloma acuminata of an antiviral composition comprising citric acid, zinc and L-arginine as active components according to the present invention when applied to the vulva of a female patient with condyloma acuminata;
FIG. 15 is a result of cytopathic effect inhibition assay of an antiviral composition comprising citric acid and/or zinc and L-arginine as active components when applied to cells infected with enterovirus; and
FIG. 16 is a result of cytopathic effect inhibition assay of an antiviral composition comprising citric acid and/or zinc and L-arginine as active components according to the present invention when applied to cells infected with poliovirus.

### BEST MODES FOR CARRYING OUT THE INVENTION

In an aspect, the present description provides an anticancer composition comprising citric acid and L-arginine as active components.

The above anticancer composition is characterized by comprising as active components citric acid in an amount of 0.01-20% by weight, preferably 0.5-5% by weight, and L-arginine in an amount of 0.01-20% by weight, preferably 0.5-10% by weight, based on the total weight of the composition, along with a pharmaceutically acceptable carrier. The carrier used in the anticancer composition includes the commonly used carriers, adjuvants and vehicles in the pharmaceutical field.

In another aspect, the present description provides an anticancer composition comprising zinc and L-arginine as active components.

The above anticancer composition is characterized by comprising as active components zinc in an amount of 0.001-5% by weight, preferably 0.01-1% by weight, and L-arginine in an amount of 0.01-20% by weight, preferably 0.5-10% by weight, based on the total weight of the composition, along with a pharmaceutically acceptable carrier. The carrier used in the anticancer composition includes the commonly used carriers, adjuvants and vehicles in the pharmaceutical field.

In a further aspect, the present invention provides uses of an anticancer composition comprising citric acid, zinc and L-arginine as active components.

The above anticancer composition is characterized by comprising as active components citric acid in an amount of 0.01-20% by weight, preferably 0.5-5% by weight, zinc in an amount of 0.001-5% by weight, preferably 0.01-1% by weight, and L-arginine in an amount of 0.01-20% by weight, preferably 0.5-10% by weight, based on the total weight of the composition, along with a pharmaceutically acceptable carrier. The carrier used in the anticancer composition includes commonly used carriers, adjuvants and vehicles in the pharmaceutical field.

Citric acid; zinc; and L-arginine, selectively used as an active component in each aspect, may be used in the anticancer compositions of the present invention in the following forms, but the present invention is not limited to them; instead, it is limited only by the claims.

Citric acid used in the present invention may be in any of the forms extracted from seeds or fruit juice of a variety of plants, where citric acid exists in a free form. Also citric acid prepared by a surface fermentation process or submerged fermentation process using the fungus *Aspergillus niger* may be used in the anticancer compositions of the present invention. In the process of preparing citric acid, fermentation is performed using molasses as an energy source, and various centrifugal separators, including a separator with self-cleaning bowl, a separator with nozzle bowl and a screw decanter, are used.

Zinc used in the present invention may be in any of the forms extracted from oysters, crustaceans, fishes, animal products such as red meat, and various vegetable products including grain, beans, nuts and seeds, in which zinc is present in abundance. Zinc may be used in various forms in the anticancer compositions of the present invention. Zinc sulfate has been used in most clinical trials, but zinc ions in other forms are also easily absorbed and utilized by the body. Examples of the available forms of zinc include chelated zinc ions with picolinate, acetate, sodium citrate, glycerate and monomethionine.

L-arginine used in the present invention may be in any of the forms prepared by fermentation or extraction from protein sources, and preferably, by a fermentation process. Methods for preparing L-arginine by fermentation may include fermentation using a microorganism resistant to arginine analogues (Agricultural Biological Chemistry, 1972, Vol. 36, p1675; Journal of General Applied Microbiology, 1973, Vol. 19, p339; Japanese Pat. Publication No. 3391-1973 and U.S. Pat. No. 3723249), L-arginine can be produced directly from carbon and nitrogen sources, for example, by using a glutaminic acid-producing microorganism belonging the genus Brevibacterium or Corynebacterium (Japanese Pat. Laid-open Publication Nos. Sho57-163487, Sho60-83593 and Sho62-265988), using an amino acid-producing microorganism with improved growth property by cell fusion (Japanese Pat. Laid-open Publication No. Sho58-158185), or using a microorganism transformed with a recombinant expression vector carrying a gene encoding an enzyme participating in the biosynthesis of L-arginine (Japanese Pat. Laid-open Publication No. Sho63-79597, Japanese Pat. Publication No. 66989/1985 and U.S. Pat. No. 4,775,623).

The anticancer composition has an activity of stimulating cell death of tumor cells or inhibiting proliferation of tumor cells by inducing apoptosis of tumor cells. In contrast to necrosis, meaning pathological cell death, apoptosis is a programmed cell death under inherent genetic control, and is induced according to an encoded program by apoptosis-related genes activated by specific external or internal factors. Activation of apoptosis-inducing genes results in biosynthesis or degradation of translation products of programmed cell death genes, eventually causing cell death. Apoptosis is typically evaluated by a biochemical method investigating DNA fragmentation, or a molecular biological method detecting expression of apoptosis-associated proteins. According to a large number of recent reports, it was demonstrated that substances inducing apoptosis in tumor cells control cell death of tumor cells, and effectively inhibit proliferation of cancers.

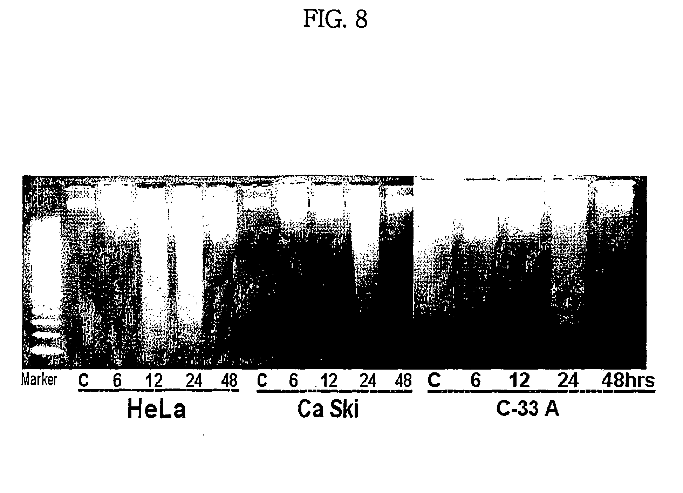

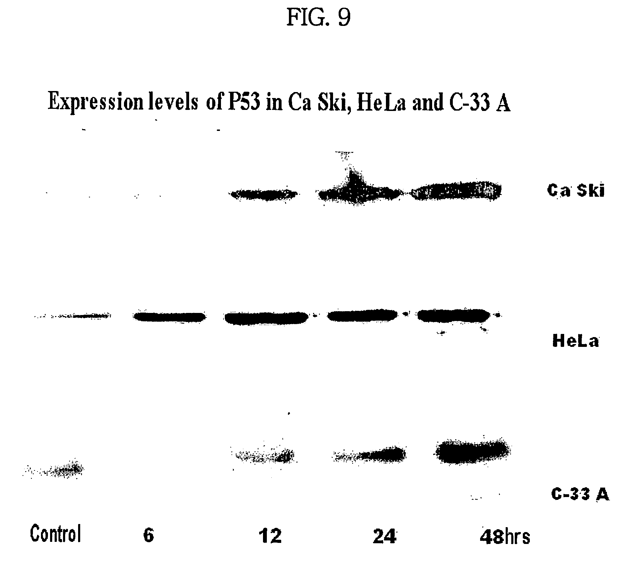

In the present invention, the aforementioned methods are used to verify that the mechanism of cell death specifically induced by the anticancer composition of the present invention is apoptosis. That is, DNA fragmentation was found in DNA samples extracted from tumor cells treated with the anticancer composition of the present invention (FIG. 8). In addition, apoptosis-associated proteins were detected in tumor cells treated with the anticancer composition of the present invention (FIGS. 9, 10 and 11). The apoptosis-associated proteins identified by the anticancer composition of the present invention are P21, P53 and C-Myc proteins. As well known in the art, expression of P53 protein leads to the synthesis of P21 protein inhibiting the activity of factors stimulating cell division. Thus, P53 and P21 proteins function to suppress cancer development by inhibiting cell division. C-Myc is an oncogenic protein, which stimulates DNA replication in tumor cells and thus activating cell division of the tumor cells.

Owing to its anticancer effect of inducing apoptosis in tumor cells, it will be apparent to those skilled in the art that the anticancer composition used in the present invention, applied to epithelial ovarian cancer as an illustrative cancer type in the present invention, is applicable to other types of cancer.

Therefore, the anticancer composition used according to the present invention may be useful for treatment of the following cancer types, but is not limited to them: bladder, breast, intestine, kidney, liver, lung (including small cell lung carcinoma), brain, esophagus, gall-bladder, ovary, pancreas, stomach, cervical, thyroid, prostate and skin (including squamous cell carcinoma) carcinomas; hematopoietic tumors in the lymphatic system including leukemia, acute lymphoid leukemia, acute lymphoblastic leukemia, B-cell lymphoma, T-cell lymphoma, Hodgkin's lymphoma, non-Hodgldn's lymphoma, hairy cell lymphoma and Burkitt's lymphoma; hematopoietic tumors in the bone marrow including acute and chronic myelogenous leukemia, myelodysplastic syndrome and promyelocytic leukemia; tumors in the central and peripheral nervous systems, including astrocytoma, neuroblastoma, glioma and schwanoma; and other tumors including melanoma, seminoma, teratoma, osteosarcoma, xeroderma pigmentosum, keratoacanthoma, thyroid follicular carcinoma and Kaposi's sarcoma.

Preferably, the anticancer composition used according to the present invention is useful for suppression or treatment of lung cancer, brain cancer, breast cancer, large intestine cancer, gestational choriocarcinoma, uterine cancer and ovarian cancer, and particularly preferably, epithelial ovarian cancer.

In a further aspect, the present description provides an antiviral composition comprising citric acid and L-arginine as active components.

The above antiviral composition is characterized by comprising as active components citric acid in an amount of 0.001-20% by weight, preferably 0.01-5% by weight, and L-arginine in an amount of 0.001-20% by weight, preferably 0.01-10% by weight, based on the total weight of the composition, along with a pharmaceutically acceptable carrier. The carrier used in the antiviral composition includes the commonly used carriers, adjuvants and vehicles in the pharmaceutical field.

In a still further aspect, the present description provides an antiviral composition comprising zinc and L-arginine as active components.

The above antiviral composition is characterized by comprising as active components zinc in an amount of 0.0001-5% by weight, preferably 0.001-1% by weight, and L-arginine in an amount of 0.001-20% by weight, preferably 0.01-10% by weight, based on the total weight of the composition, along with a pharmaceutically acceptable carrier. The carrier used in the antiviral composition includes the commonly used carriers, adjuvants and vehicles in the pharmaceutical field.

In a still further aspect, the present invention provides uses of an antiviral composition comprising citric acid, zinc and L-arginine as active components.

The above antiviral composition is characterized by comprising as active components citric acid in an amount of 0.001-20% by weight, preferably 0.01-5% by weight, zinc in an amount of 0.0001-5% by weight, preferably 0.001-1% by weight, and L-arginine in an amount of 0.001-20% by weight, preferably 0.01-10% by weight, based on the total weight of the composition, along with a pharmaceutically acceptable carrier. The carrier used in the antiviral composition includes the commonly used carriers, adjuvants and vehicles in the pharmaceutical field.

The activity of the antiviral composition used in the present invention may be verified by analyzing expression of viral proteins in cancer cell lines. That is, after a cancer cell line is treated with the present antiviral composition, proteins are isolated from the cell line and analyzed for presence of viral proteins by a molecular biology technique commonly known in the art. In addition, the activity of the present antiviral composition may be verified by confirming viral infections to be prevented or treated when directly applied to viral infection sites in the body.

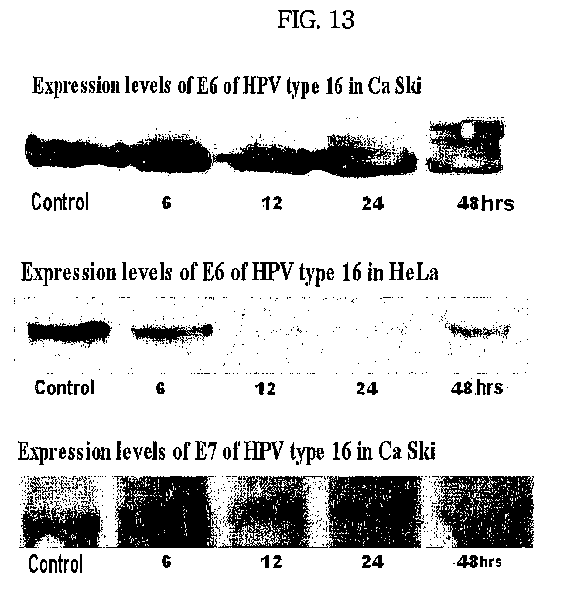

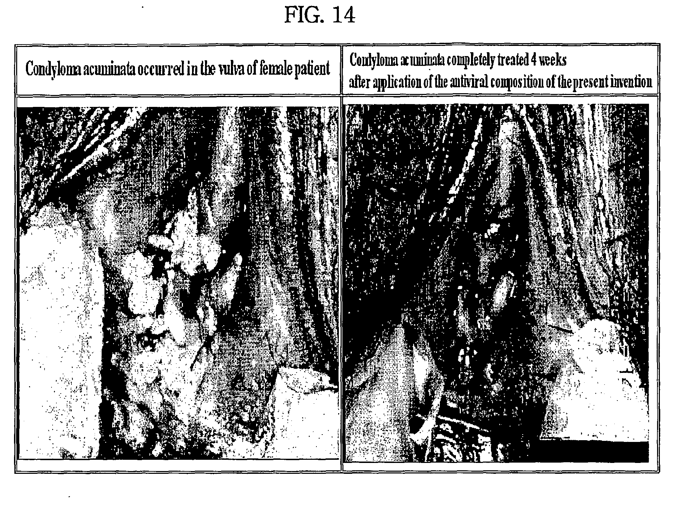

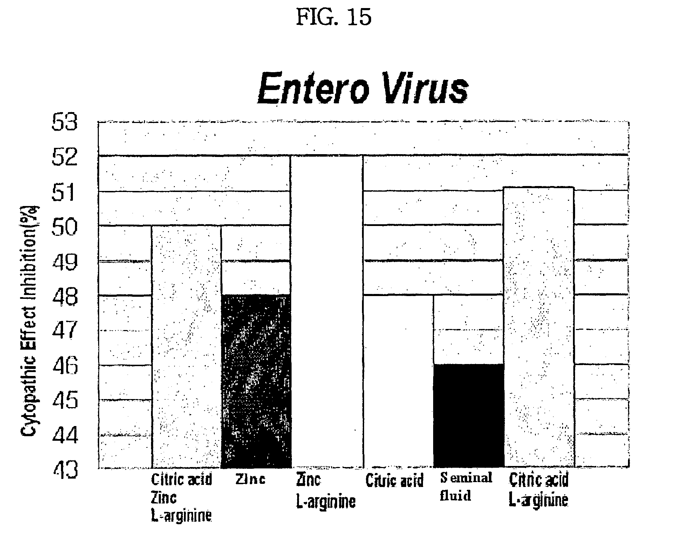

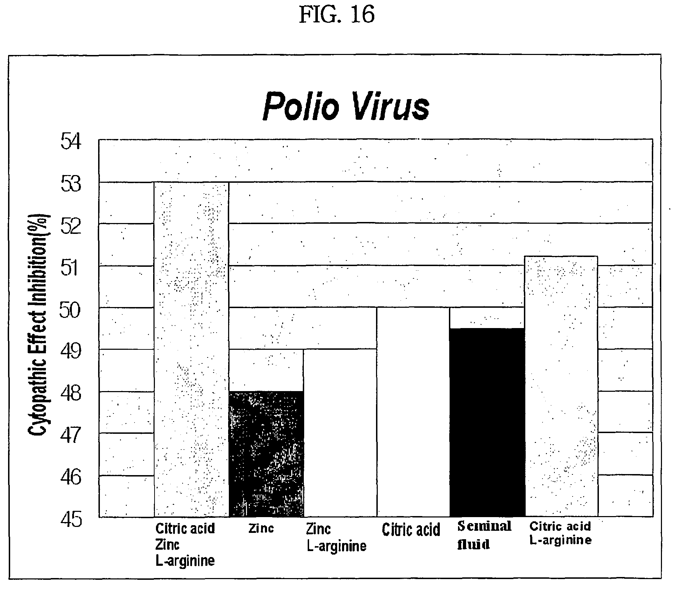

According to the embodiment of the present invention, among papillomavirus proteins, E6 and E7 proteins of HPV type 16 or 18 were analyzed, which are known as antigenic proteins causing uterine cancer. In this embodiment, the present antiviral composition is demonstrated to reduce the expression of the E6 and E7 proteins in a cervical cancer cell line (FIG. 13). Also, genital warts were found to be treated when the present antiviral composition was applied to the vulva of a female patient with genital warts by HPV infections (FIG. 14). Further, when cells infected with enterovirus or poliovirus were treated with the present antiviral composition, cytopathic effect (CPE) did not occur by the inhibition of viral proliferation (FIGS. 15 and 16).

The antiviral composition used according to the present invention may be used for preventing or treating diverse diseases caused by viral infections. Therefore, non-limiting examples of the diseases caused by viral infections, which can be treated or prevented by use of the present antiviral composition, include warts that are skin growths caused by infection with papillomavirus, which stimulates the local proliferation of cells in the epidermis of the skin and thus leads to enlargement of the keratin layer, and condyloma acuminata caused by papillomavirus infections around genital organs or the anus. In addition, the present antiviral composition has effects of inhibiting viral proliferation, and thus may inhibit proliferation of AIDS virus causing AIDS (Acquired Immune Deficiency Syndrome), influenza virus, enterovirus causing meningitis, poliovirus causing infantile paralysis, etc.

In a preferred aspect, the antiviral composition used according to the present invention may prevent or treat diseases caused by papillomavirus, AIDS virus, enterovirus or poliovirus.

The carrier used in the anticancer or antiviral composition for used in the present invention (hereinafter, referred to as "the present composition") includes the commonly used carriers, adjuvants and vehicles, in the pharmaceutical field, which are as a whole called "pharmaceutically acceptable carriers". Non-limiting pharmaceutically acceptable carriers used in the anticancer composition of the present invention include ion exchange, alumina, aluminum stearate, lecithin, serum proteins, buffering agents (e.g., sodium phosphate, glycine, sorbic acid, potassium sorbate, partial glyceride mixtures of vegetable saturated fatty acids), water, salts or electrolytes (e.g., protamine sulfate, disodium hydrophosphate, potassium hydrophoshate, sodium chloride, and zinc salts), colloidal silica, magnesium trisilicate, polyvinylpyrrolidone, cellulose-based substrates, polyethylene glycol, sodium carboxymethylcellulose, polyarylate, waxes, polyethylene-polyoxypropylene-block copolymers, polyethylene glycol, and wool fat.

The present composition may be administered via any of the common routes, if being able to reach a desired tissue. Therefore, the present composition may be administered topically, orally, parenterally, intraocularly, transdermally, intrarectally and intraluminally, and may be formulated into solutions, suspensions, tablets, pills, capsules, sustained release preparations, ointments, creams, and the like. The term "parenteral", as used herein, includes subcutaneous, intranasal, intravenous, intraperitoneal, intramuscular, intra-articular, intra-synovial, intrasternal, intracardial, intrathecal, intralesional and intracranial injection or infusion techniques.

In an aspect, when formulated as a solid preparation for oral application, the present composition may include, in addition to the active ingredient, diluents (e.g., lactose, dextrose, sucrose, cellulose, corn starch or potato starch), lubricants (e.g., silica, talc, stearic acid, magnesium or calcium stearate, and/or polyethylene glycol), binders (e.g., starch, gum arabic, gelatin methylcellulose, carboxymethylcellulose or polyvinyl pyrrolidone), disintegrators (e.g., starch, alginic acid, alginate or sodium starch glycolate), formal mixtures, dyes, sweetening agents, humectants (e.g., lecithin, polysorbate, laurylsulfate), and the commonly used, pharmaceutically inert substances. The solid formulation for oral administration may be prepared by the methods known in the art, for example, via a process comprising mixing, granulation, tableting and sugar-coating or film-coating.

In another aspect, when formulated as liquid compositions for oral application, such as solutions, emulsions, suspensions, syrups or elixirs, the present composition may include the commonly used inert diluents (e.g., purified water, ethanol). If desired, the liquid composition may further include adjuvants, for example, humectants and emulsifiers, sweetening agents, perfumes, aromatic agents, and antiseptic agents.

In a further aspect, the present composition may be formulated as aqueous solutions for parenteral administration. Preferably, a suitable buffer solution, such as Hank's solution, Ringer's solution or physiologically buffered saline, may be employed. Aqueous injection suspensions may be supplemented with substances capable of increasing viscosity of the suspensions, which are exemplified by sodium carboxymethylcellulose, sorbitol and dextran. In addition, suspensions of the active components, such as oily injection suspension, include lipophilic solvents or carriers, which are exemplified by fatty oils such as sesame oil, and synthetic fatty acid esters such as ethyl oleate, triglycerides or liposomes. Polycationic non-lipid amino polymers may be also used as vehicles. Optionally, the suspensions may contain suitable stabilizers or drugs to increase the solubility of components and obtain high concentrations of the components.

In still another aspect, the present composition is preferably in the form of a sterile injectable preparation, such as a sterile injectable aqueous or oleaginous suspension. Such suspension may be formulated according to the methods known in the art, using suitable dispersing or wetting agents (e.g., Tween 80) and suspending agents. The sterile injectable preparations may also be a sterile injectable solution or suspension in a non-toxic parenteially-acceptable diluent or solvent, such as a solution in 1,3-butanediol. The acceptable vehicles and solvents include mannitol, water, Ringer's solution and isotonic sodium chloride solution. In addition, sterile fixed oils may conventionally be employed as a solvent or suspending medium. For this purpose, any bland fixed oil may be employed, including synthetic mono- or diglycerides. In addition, fatty acids, such as oleic acid and glyceride derivatives thereof, may be used in the preparation of injectable preparations, likewise the pharmaceutically acceptable natural oils (e.g., olive oil or castor oil), and particularly, polyoxyethylated derivatives thereof.

The aforementioned aqueous composition is sterilized mainly by filtration using a filter to remove bacteria, mixing with disinfectants or in combination with radiation. The sterilized composition can be hardened, for example, by freeze-drying to obtain a hardened product, and for practical use, the hardened composition is dissolved in sterilized water or a sterilized diluted solution.

In still another aspect, the antiviral composition for use in the present invention is preferably in the formulations for transdermal administration, which comprises therapeutically effective amounts of the active components along with a pharmaceutically acceptable carrier. The term "transdermal administration" means that a therapeutically effective amount of an active component contained in a pharmaceutical composition transmits into the skin when the pharmaceutical composition is topically applied to the skin.

The transdermal formulations obtainable according to the present invention include creams, ointments, lotions, gels, external solutions, pastes, liniments, external powders, aerosols and trandermal absorbents. These formulations are described in the literature that is a guidebook generally known in all pharmaceutical chemistry fields (Remington's Pharmaceutical Science, 15th Editions, 1975, Mack Publishing Company, Easton, Pennsylvania, 18042, Chapter 87: Blaug, Seymour).

Representatively, the creams obtainable by the present invention include cold creams, emollient creams, shaving creams, vanishing creams and hand creams. Preferable are vanishing creams, which are oil in water (o/w) types typically containing stearic acid and water. When the vanishing creams are applied to the skin, water is evaporated and a thin layer of stearic acid is formed. In addition, the cold creams, which may be suitably used, are semi-solid white water in oil (w/o) types that are composed of cetyl alcohol, bee wax, white wax, sodium borate and distilled water. The ointments obtainable by the present invention are typically classified into hydrocarbon bases, absorption bases, water-removable bases and water-soluble bases, and all types of the ointments are well known to those skilled in the pharmaceutical formulation fields and included in the present invention. The lotions of the present invention are typically classified into suspensions and emulsions, and typically prepared using an emulsifier or another suitable stabilizer. All types of the lotions are well known to those skilled in the pharmaceutical formulation fields, and included in the present invention.

With reference to the above description, as illustrative examples of the transdermal formulations obtainable according to the present invention, creams, ointments, gels and pastes will be described, as follows.

As a representative example of the transdermal formulations, a cream is prepared, as follows. An oil phase and an aqueous phase are separately heated to 65 to 75°C, where the oil phase is composed of alcohols, bee wax, sorbitan monooleate, etc., and the aqueous phase is composed of a sorbitol solution, polysorbate, methylparabene, propylparabene, glycerin, potassium hyroxide, distilled water, etc. The oil phase is slowly added to the aqueous phase with stirring to generate a crude emulsion. The emulsion is homogenized by cooling it to a temperature at which volatilization and degradation does not occur, and stirred at a prescribed rate until coagulation.

The ointments may be prepared on a large or small scale. When the ointments are prepared by a particular formulation method, the method is determined greatly depending on physical and chemical properties of the components of the ointments. When the ointments are prepared on a small scale, their components may be mixed by incorporation using several methods. In case of large-scale preparation, a melting method may be used, with which all or a part of the components of the ointments are melted and then cooled with stirring at a prescribed rate until coagulation. Herein, undissolved components generally require a mixing step together with cooling. Typically, the ointments are prepared within low temperatures at which heat-unstable or volatile substances are not degraded or volatile.

The gel may be prepared as follows. Metocel is dispersed in distilled water preheated to 80 to 90°C. After 12 hrs of incubation, a gel composition is dispersed in the distilled water to generate a dispersion solution. Then, a sodium hydroxide solution is added to the dispersion solution, and the solution is adjusted to neutral pH. The resulting solution is mixed with methylparabene, metocel, carbopol, propylene glycol, etc., to give a gel.

The paste may be made by pulverizing a paste composition in a suitable container such as a mortar and homogenously mixing the pulverized product with Vaseline, etc.

Transdermal absorption of citric acid, zinc and L-arginine, which are active components in the transdermal formulations obtainable by the present invention, may be stimulated by the known physical and chemical methods. Suitable are physical methods employing mainly heat, electrical and ultrasonic energy, but chemical methods are preferable. Transdermal absorption stimulators usable in the chemical methods according to the present invention include those listed in Table 1, below.

**TABLE 1**

| | | |
|---|---|---|
| Representative transdermal absorption stimulators and their types | | |

| Types of chemical absorption stimulators | | Compounds |
|---|---|---|
| Surfactants | Non-ionic | Tweens, Brij, spans, Triton X-100, polozymer |
| | Anionic | Sodium lauryl sulfide |
| | Cationic | N,N-bis(2-hydroxyethyl)oleylamine |
| Solvents | Sulfoxide | DMSO, dodecylmethyl sulfoxide (DCMS), DMF |
| | Exocyrate | Dodecanol, hexanesocylate |
| | Alcohols | Ethanol |
| | Polyolice | Propylene glycol, polyethylene glycol, glycerine |
| | Pyrolidones | PVP, 2-pyrolidone, N-methyl-2-pyrolidone |
| Fats | Fatty acids | Oleic acid, linoleic acid, laurinic acid, myristinic acid, stearic acid, IPM, IPP, carnic acid, caprinic acid |
| | Aliphatic alcohols | Oleyl, lauryl, steraryl |
| Zwitterionic | | Dodecyldimethylaminopropane sulfate |
| Amide | | n,n-diethyl-m-toluamide (DEET) |
| Ureas | | Urea, 1-dodecylurea, 1,3-didodecylurea, 1,3-diphenylurea |
| Terpene | | Ucaliptol, mentol, limone oxide |
| Lactams | | Laurocapram (azone) |
| Cheratose | | Disodium DETA |
| Bile salt | | Glycocolate, calcium-thioglycholate |
| Macrosalicinic | | Macrosalicinic ketone/lactone |

The term "therapeutically effective amount", as used herein in connection with the present composition, means an amount at which an active component shows improved or therapeutic effect toward a cancer type treated with the present composition. The therapeutically effective amount of the present composition may vary according to patient's age and sex, application sites, administration frequency, administration duration, formulation type and adjuvant types. Typically, in case of being in the form of injection preparations, the present composition is administered in an amount of 50 to 5000 mg, preferably, 100 to 4000 mg, and most preferably, 250 to 3000 mg, over 1 to 5 times per day. In case of being in the form of orally administered preparations, the present composition is typically administered in an amount of 1 to 2,000 mg, preferably, 250 to 1,000 mg, and most preferably, 300 to 500 mg, over 1 to 5 times per day. In addition, in case of being in the form of transdermal preparations, to obtain preventory or therapeutic effect, the present composition is typically administered in an amount of 500 to 2000 mg once or twice per day for about two to three weelcs.

The present invention will be explained in more detail with reference to the following examples (not necessarily according to the invention) in conjunction with the accompanying drawings. However, the following examples are provided only to illustrate the present invention, and the present invention is not limited to the examples.

### EXAMPLE 1: Preparation of injection preparations

The compositions for use according to the present invention, comprising various combinations of active components, were prepared as follows. Citric acid (Sigma, USA), zinc (Sigma, USA), and L-arginine (Sigma, USA) were selectively mixed according to the mixing ratios in Table 2, below, and each of the mixtures was dissolved in sterilized water. The resulting mixture was then filled into a vial (100 mg).

**TABLE 2**

| Contents (wt%) of active components of the compositions for use according to the present invention | | | |
|---|---|---|---|
| Composition | Citric acid | Zinc | L-arginine |
| 1 | 4 | 0 | 12 |
| 2 | 0 | 0.2 | 6 |
| 3 | 2 | 0.2 | 6 |

### EXAMPLE 2: Preparation of tablets

Citric acid (Sigma, USA), zinc (Sigma, USA), and L-arginine (Sigma, USA) were selectively mixed according to the mixing ratios in Table 2, above, and each of the mixtures was mixed with 30 wt% of lactose, 5 wt% of magnesium stearate, 10 wt% of sodium starch glycolate, and sterilized water. The resulting mixture was incubated at 30-60°C for 1 hr with stirring, and cooled to room temperature. Thereafter, the mixture was tableted according to the conventional methods, thereby producing tablets each containing 350 mg of the powdered mixture.

### EXAMPLE 3: Preparation of transdermal formulations

The antiviral composition obtainable by the present invention was formulated into transdermal forms, as follows. 2 wt% of citric acid, 0.1 wt% of zinc and 5 wt% of L-arginine were dissolved 1 L ethanol contained in a beaker, and placed at -20°C for 24 hrs. Then, the generated coagulation was recovered and used as an active ingredient of the transdermal formulations. The coagulation is an about ten-fold concentrate.

### (1) Preparation of cream

| Oil phase | |
|---|---|
| Stearic acid | 13 wt% |
| Stearyl acohol | 1 wt% |
| Ethyl alcohol | 1 wt% |
| The prepared coagulation | 5 wt% |

| Aqueous phase | |
|---|---|
| Glycerin | 10 wt% |
| Methylparabene | 0.1 wt% |
| Propylparabene | 0.05 wt% |
| Potassium hydroxide | 0.9 wt% |
| Purified water | up to 100 wt% |

An oil phase and an aqueous phase, each which was prepared according to the above composition, were separately heated to 65°C. Then, the oil phase was slowly added to the aqueous phase with stirring to generate a crude emulsion. The emulsion was cooled with agitation until coagulation, thus giving a cream.

### (2) Preparation of ointment

| | |
|---|---|
| Vaseline | 80 wt% |
| Stearyl alcohol | 3 wt% |
| White wax | 9 wt% |
| Cholesterol | 3 wt% |
| The prepared coagulation | 5 wt% |

Stearyl alcohol, white wax, cholesterol and the prepared coagulation were melted in a steam bath. After adding Vaseline to the mixture, the resulting mixture was slowly heated until a liquid solution was obtained. Then, the liquid solution was cooled with stirring until coagulation, thus giving an ointment.

### (3) Preparation of gel

| | |
|---|---|
| Metocel 90 H.C. 4000 | 0.8 wt% |
| Carbopol 934 | 0.24 wt% |
| Propylene glycol | 16.7 wt% |
| Methylparabene | 0.015 wt% |
| The prepared coagulation | 5 wt% |
| Sodium hydroxide | amount required for adjustment to pH 7.0 |
| Purified water | up to 100 wt% |

Metocel was dispersed in hot water (80 to 90 °C), and cooled in a refrigerator overnight to generate a liquid solution. Separately, carbopol 934 and the coagulation were dispersed in water, adjusted to pH 7.0 with a sufficient amount of sodium hydroxide solution, and then supplemented with water to give a final volume of 40 ml. Also, separately, methylparabene was dissolved in propylene glycol. The three solutions were mixed to generate a gel.

### (4) Preparation of paste

| | |
|---|---|
| Oxidized iron | 25 wt% |
| Starch | 25 wt% |
| Kelamin | 5 wt% |
| The prepared coagulation | 5 wt% |
| Vaseline | up to 100 wt% |

Kelamin was titrated with oxidized iron, the coagulation and starch, pulverized in a mortar, and homogeneously mixed with Vaseline, thus yielding a paste.

### EXAMPLE 4: Evaluation of effects of anticancer composition comprising citric acid and L-arginine as active components on viability of tumor cells

The anticancer composition comprising citric acid and L-arginine as active components was evaluated for its effect on growth and survival of tumor cells, by preparing several compositions comprising various concentrations of citric acid according to Table 3, below, treating tumor cells with the compositions, and investigating cellular viability. SKOV-3 cells (human ovarian adenocarcinoma cell line (derived from malignant ascites), ATCC No. HTB-77) and U87 (human glioblastoma cell line, ATCC No. HTB-14) were used.

SKOV-3 and U-87 cells were plated onto 96-well plates at a density of 3×10³ cells, and cultured for 12 hrs in DMEM (Dulbecco's modified Eagle's medium, Life Technology, Inc., U.S.A.) supplemented with 10% (v/v) FBS (fetal bovine serum), 100 µg/ml of streptomycin, 100 U/ml of penicillin and 100 µg/ml of L-glutamine.

The cultured cells were treated with the compositions comprising various concentrations of citric acid and L-arginine, which were prepared according to the same method as in Example 1, and incubated at 37°C under 5% CO₂ for 24 hrs. Thereafter, cellular viability was evaluated.

**TABLE 3**

| | | | | | | | | |
|---|---|---|---|---|---|---|---|---|
| Contents (wt%) of citric acid and L-arginine of the compositions | | | | | | | | |

| Composition No. | Control | 1 | 2 | 3 | 4 | 5 | 6 | 7 |
|---|---|---|---|---|---|---|---|---|
| Conc. of citric acid | 0 | 0.2 | 0.4 | 0.8 | 1 | 1.5 | 2 | 4 |
| Conc. of L-arginine | 0 | 0.6 | 1.2 | 2.4 | 3 | 4.5 | 6 | 12 |

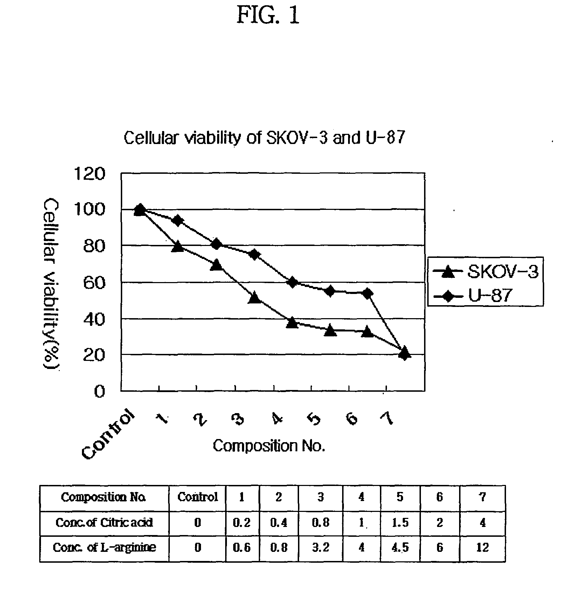

Cellular viability was analyzed by the known MTT (3-(4,5 dimethylthiazol-2-yl)-2,5-2H-tetrazolium bromide) assay (Hansen, M.B. et al., J. Immunol. Methods, 172, 203-210 (1989)). 20 µl of an MTT solution (10 mg/ml of MTT in PBS (phosphate buffered saline) was added to each well, and the plates were incubated at 37°C for 4 hrs. After removing the culture medium, formazan crystal dissolved in 200 µl of DMSO (dimethyl sulfoxide) was added to each well, followed by incubation at room temperature for 10 min with agitation. Absorbance was measured at 540 nm using a Bio-Rad model 3550 microplate reader (Richmond, CA.). Herein, the cells not treated with the composition comprising citric acid were used as a control. The results are given in FIG. 1.

As shown in FIG. 1, when treating the tumor cell lines SKOV-3 (▲) and U-87 (◆) with the compositions comprising citric acid and L-arginine as active components, cellular viability was found to be inversely dependent on concentration of the active components, citric acid and L-arginine. In particular, in case of being treated with the composition 5, viability of the tumor cells was reduced to below 60%, while being reduced to below 20% in case of being treated with the composition 7.

### EXAMPLE 5: Evaluation of effects of anticancer composition comprising zinc and L-arginine as active components on viability of tumor cells

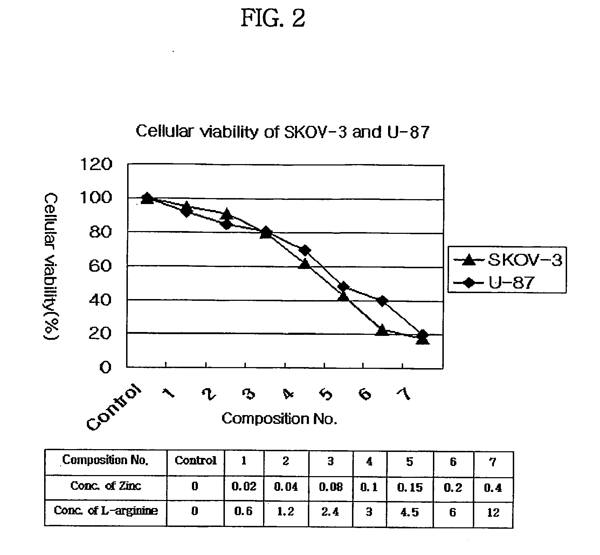

The anticancer composition comprising zinc and L-arginine as active components was evaluated for its effects on growth and survival of tumor cells, by preparing several compositions comprising various concentrations of zinc and L-arginine according to Table 4, below, treating tumor cells with the compositions, and investigating cellular viability. SKOV-3 and U-87 cell lines were used. Cell culture, treatment of the tumor cells with the compositions and cellular viability analysis were performed according to the same method as in Example 4. The results are given in FIG. 2.

**TABLE 4**

| | | | | | | | | |
|---|---|---|---|---|---|---|---|---|
| Contents (wt%) of zinc and L-arginine of the compositions | | | | | | | | |

| Composition No. | Control | 1 | 2 | 3 | 4 | 5 | 6 | 7 |
|---|---|---|---|---|---|---|---|---|
| Conc. of zinc | 0 | 0.02 | 0.04 | 0.08 | 0.1 | 0.15 | 0.2 | 0.4 |
| Conc. of L-arginine | 0 | 0.6 | 1.2 | 2.4 | 3 | 4.5 | 6 | 12 |

As shown in FIG. 2, when treating the tumor cell lines SKOV-3 (▲) and U-87 (◆) with the compositions comprising zinc and L-arginine as active components, cellular viability was found to decrease in a manner inversely dependent on concentration of the active components, zinc and L-arginine. In particular, in case of being treated with the composition 5, viability of the tumor cells were reduced to below 50%, while being reduced to below 20% in case of being treated with the composition 7.

### EXAMPLE 6: Evaluation of effects of anticancer composition comprising citric acid, zinc and L-arginine active components on viability of tumor cells

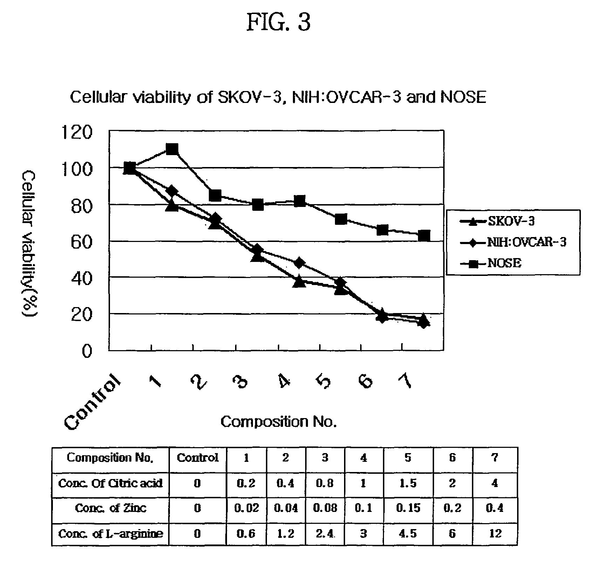

The anticancer composition comprising citric acid, zinc and L-arginine as active components was evaluated for its effects on growth and survival of tumor cells, by preparing several compositions comprising various concentrations of citric acid, zinc and L-arginine according to Table 5, below, treating tumor cells with the compositions, and investigating cellular viability. SKOV-3, NIH:OVCAR-3 (human ovarian adenocarcinoma cell line, ATCC No. HTB-161) and NOSE (human ovarian surface normal epithelial cell line) were used. Cell culture, treatment of the tumor cells with the compositions and cellular viability analysis were performed according to the same method as in Example 4. The results are given in FIG. 3.

**TABLE 5**

| | | | | | | | | |
|---|---|---|---|---|---|---|---|---|
| Contents (wt%) of citric acid, zinc and L-arginine of the compositions | | | | | | | | |

| Composition No. | Control | 1 | 2 | 3 | 4 | 5 | 6 | 7 |
|---|---|---|---|---|---|---|---|---|
| Conc. of citric acid | 0 | 0.2 | 0.4 | 0.8 | 1 | 1.5 | 2 | 4 |
| Conc. of zinc | 0 | 0.02 | 0.04 | 0.08 | 0.1 | 0.15 | 0.2 | 0.4 |
| Conc. of L-arginine | 0 | 0.6 | 1.2 | 2.4 | 3 | 4.5 | 6 | 12 |

As shown in FIG. 3, when treating the tumor cell lines SKOV-3 (▲), NIH:OVCAR-3 (◆) and NOSE (■) with the compositions comprising citric acid, zinc and L-arginine as active components, cellular viability was found to decrease in a manner inversely dependent on concentration of the active components, citric acid, zinc and L-arginine. In particular, in case of being treated with the composition 6, viability of the SKOV-3 and NIH:OVCAR-3 cells was reduced to below 20%. In contrast, when NOSE cells were treated with the compositons 6 and 7, cellular viability was found to remain over 50%. These results indicate that the anticancer compositions act specifically to tumor cells and finally induce cell death of the tumor cells.

### EXAMPLE 7: Evaluation of effects of anticancer composition comprising citric acid, zinc and L-arginine as active components on viability of tumor cells

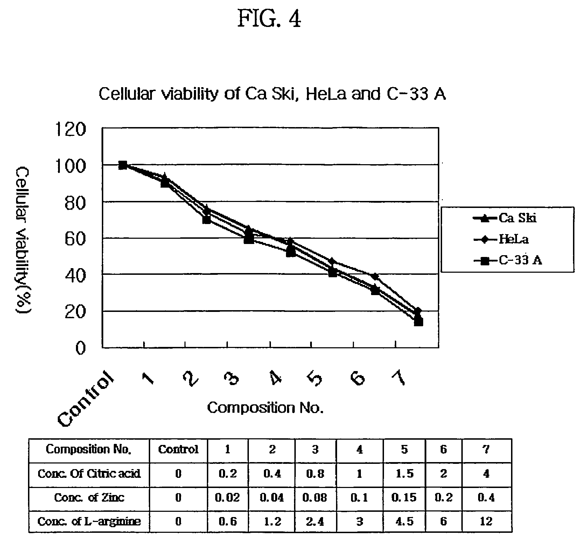

The anticancer composition comprising citric acid, zinc and albumin as active components was evaluated for its effects on growth and survival of tumor cells, using several compositions prepared in the Example 5, which comprise various concentrations of citric acid, zinc and albumin according to the above Table 5, by treating tumor cells with the compositions and investigating cellular viability. Ca Ski (ATCC No. CRL-1550), HeLa (ATCC No. CCL-2) and C-33 (ATCC No. HTB-31) were used. Cell culture, treatment of the cells with the compositions and cellular viability analysis were performed according to the same method as in Example 4. The results are given in FIG. 4.

As shown in FIG. 4, when treating Ca Ski (▲), HeLa (◆) and C-33 A (■) cells with the compositions comprising citric acid, zinc and L-arginine as active components, viability of the tumor cells was found to decrease in a manner inversely dependent on concentration of the active components citric acid, zinc and L-arginine. In particular, in case of being treated with the composition 5, viability of the tumor cells was reduced to below 60%, while being reduced to below 20% in case of being treated with the composition 7.

### EXAMPLE 8: Flow cytometric analysis of cells treated with the anticancer composition obtainable by of the present invention

The anticancer composition obtainable by the present invention, prepared in the Example 1, was evaluated for its effects on DNA division by flow cytometry.

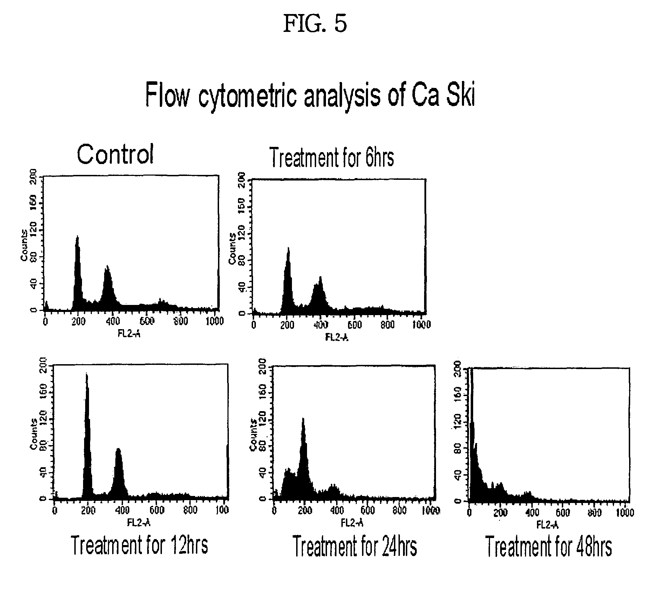

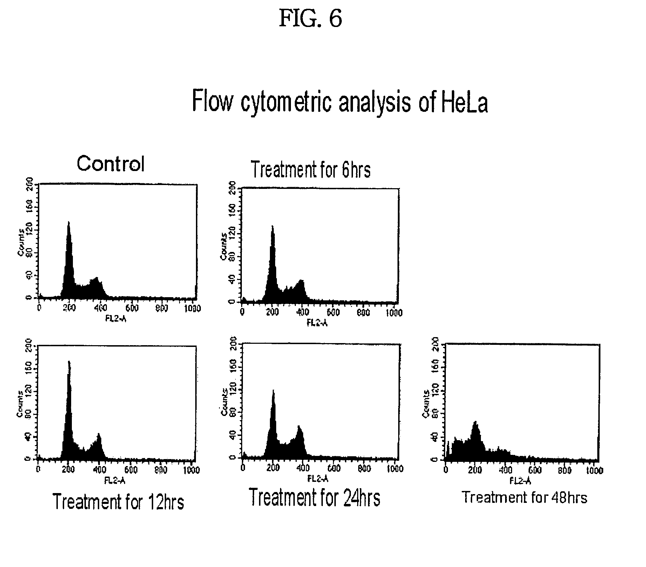

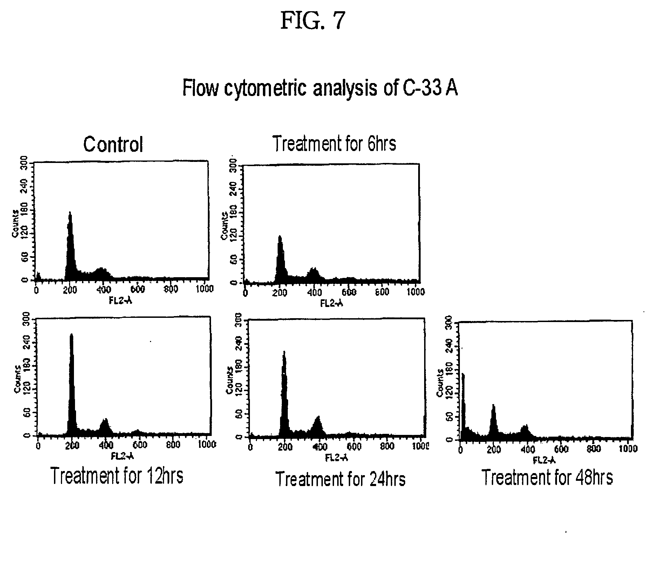

WI 38, OVCAR-3 and SK-OV-3 cells were plated onto 96-well plates at a density of 2x10⁶ cells, 100 µl of the anticancer composition prepared in Example 1 (the anticancer composition 3 of Table 1) was added to each well, and the plates were incubated for 6 hrs and 24 hrs according to the same method as in Example 4. A control was not treated with the anticancer composition. Cells were harvested, and washed with pre-cooled PBS twice. The washed cells were fixed with 100% ethanol at 4°C for 24 hrs. The resulting cells were suspended in 500 µl of PBS, treated with 20 µg/ml of RNase at 37°C for 30 min, and cooled on ice for 10 min. The resulting isolated DNA was stained with 50 µg/µl of PI (propidium iodide), and DNA division was analyzed using a flow cytometer (Becton Dickinson FACS system, U.S.A.). Herein, propidium fluorescence was developed using a cooled 15 mW argon laser, and emitted light was collected using a 617 long pass optical filter. Cell cycle distribution was determined by ModFit software (ModFit, Verity Software House, Topsham, ME, U.S.A.). The results are given in FIGS. 5, 6 and 7.

As shown in FIGS. 5, 6 and 7, in Ca Ski, HeLa and C-33 A cell lines treated with the anticancer composition of the present invention, the percentage of cells was reduced in the G₁ phase (DNA synthesis). In addition, cells were accumulated in the G₀-G₁ (sub-G₁ fraction) phase, while cell population was significantly reduced in the cell cycle phases S (DNA synthesis) and G2-M (mitosis), indicating induction of apoptosis.

### EXAMPLE 9: DNA laddering assay of cells treated with the anticancer composition obtainable by of the present invention

DNA laddering assay was performed to investigate the mechanism of cell death induced by the anticancer composition of the present invention, observed in Example 8. Ca Ski, HeLa and C-33 A cells were plated onto 96-well plates at a density of 2×10⁶ cells, 100 µl of the anticancer composition prepared in the Example 1 (the anticancer composition 3 of Table 1) was added to each well, and the plates were incubated for 6, 12, 24 and 48 hrs according to the same method as in Example 4. Cells were harvested individually after the incubation, and DNA was then isolated by the conventionally known method, as follows (Leszczynski D. et. al. Photochem. Photobiol., 1996, Vol. 64, p936-.). The harvested cells were suspended in lysis buffer consisting of 0.5% SDS (sodium-dodecyl-sulfate), 2 mM EDTA (ethylene diamine tetra acetic acid), 100 mg/ml of proteinase K and 50 mM Tris-HCl buffer (pH 8.0), incubated at 55°C for 3 hrs, and extracted with an equal volume of phenol: chloroform: isoarnylalcohol (25:24:1). Then, DNA was precipitated with 0.1 volume of ammonium acetate and 2.5 volume of cool absolute ethanol, followed by incubation at -20°C overnight. DNA pellet was dissolved in TE buffer (0.5 M EDTA in 1 M Tris-HCl buffer, pH 8.0), and treated with 100 mg/ml of RNase A at 37°C for 1hr. The resulting DNA sample was separated on a 1.5% agarose gel containing 0.5 µg/ml of ethidium bromide under 60 V for 1 hr, and exposed to UV. The results are given in FIG. 8.

As shown in FIG. 8, in Ca Ski, HeLa and C-33 A cells treated with the anticancer composition according to the present invention, about 100-bp DNA ladder was observed 12 and 24 hrs after treatment. Typically, nucleosomes are cleaved at linker regions between nucleosomal units during induction of apoptosis, resulting in production of about 100-bp DNA ladder. These results indicate that the mechanism of cell death induced by the anticancer composition obtainable by the present invention is apoptosis.

### EXAMPLE 10: Expression analysis of apoptosis-associated and oncogenic proteins in cells treated with the anticancer composition according to the present invention

Expression patterns of the apoptosis-associated proteins P53 and P21 and the oncogenic protein C-Myc were analyzed to investigate the mechanism of cell death induced by the anticancer composition of the present invention.

Ca Ski, HeLa and C-33 A cells were plated onto 96-well plates at a density of 2×10⁶ cells, 100 µl of the anticancer composition prepared in Example 1 (the anticancer composition 3 of Table 1) was added to each well, and the plates were incubated for 30 min, 6, 12, 24 and 48 hrs according to the same method as in Example 4. Cells were harvested individually after the incubation, and lysed with lysis buffer (10mM Tris, 1mM EDTA, 1mM DTT, 1mM PMSF, protease inhibitor). The resulting cell lysate was subjected to SDS-electrophoresis, and the separated protein was transferred to an ECL nitrocellulose membrane (Amersham Life Science, U.K.). The membrane was then blocked with a blocking solution (5% skim milk in TBST; 10mM Tris-HCl, pH 8.0. 150 mM NaCl, 0.1% Tween 20) at 4°C overnight Thereafter, apoptosis-associated proteins were investigated by immunoblotting analysis, as follows. Primary antibodies to P21, P53 and C-Mycwere diluted to 1:500 in the blocking solution. The blocked membrane was reacted with the diluted primary antibodies at 4°C overnight, and then with a 1:5000 dilution of a secondary antibody (goat anti-rabbit IgG-HRP; Santa Cruz Biotechnology, USA) in the blocking solution at room temperature for 1 hr. After each treatment of primary and secondary antibodies, the membrane was washed three times for 15 min per each washing, and exposed to an ECL Hyperfilm (Amersham Life Science, U.K.). The results are given in FIGS. 9,10 and 11.

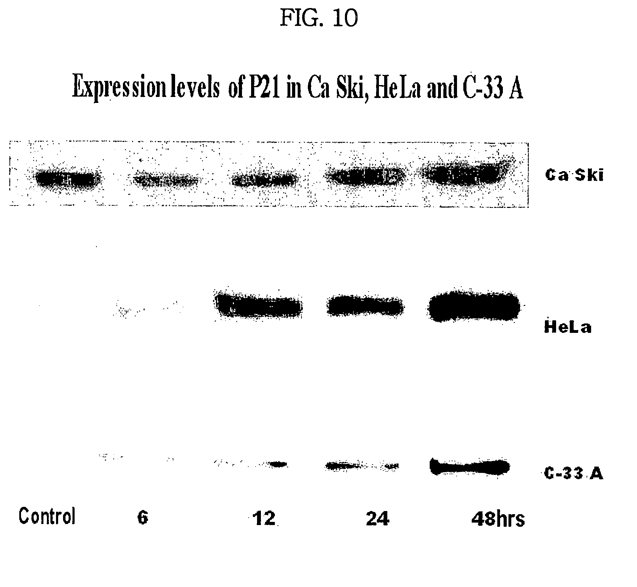

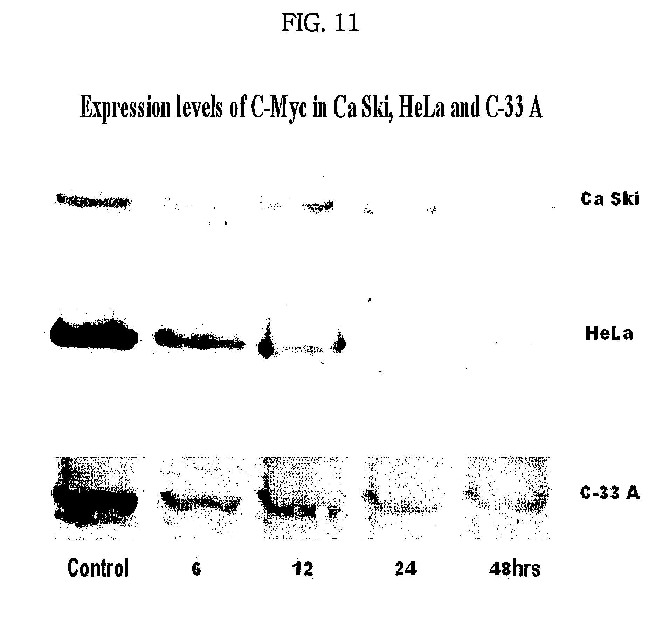

As shown in FIGS. 9 and 10, in cells treated with the anticancer composition obtainable by the present invention, expression of P21 and P53 proteins suppressing cell division was gradually increased with the passage of time. On the other hand, as shown in FIG. 11, in cells treated with the anticancer composition obtainable by the present invention, expression of C-Myc protein activating cell division of tumor cells was gradually reduced with the passage of time. These results indicate that the anticancer composition obtainable by the present invention suppresses proliferation of tumor cells and eventually induces apoptosis of tumor cells.

### EXAMPLE 11: Nuclear staining of cells treated with the anticancer composition obtainable by the present invention

To investigate the mechanism of cell death induced by the anticancer composition obtainable by the present invention, observed in Example 8, nuclear staining was performed in cells treated with the anticancer composition obtainable by the present invention.

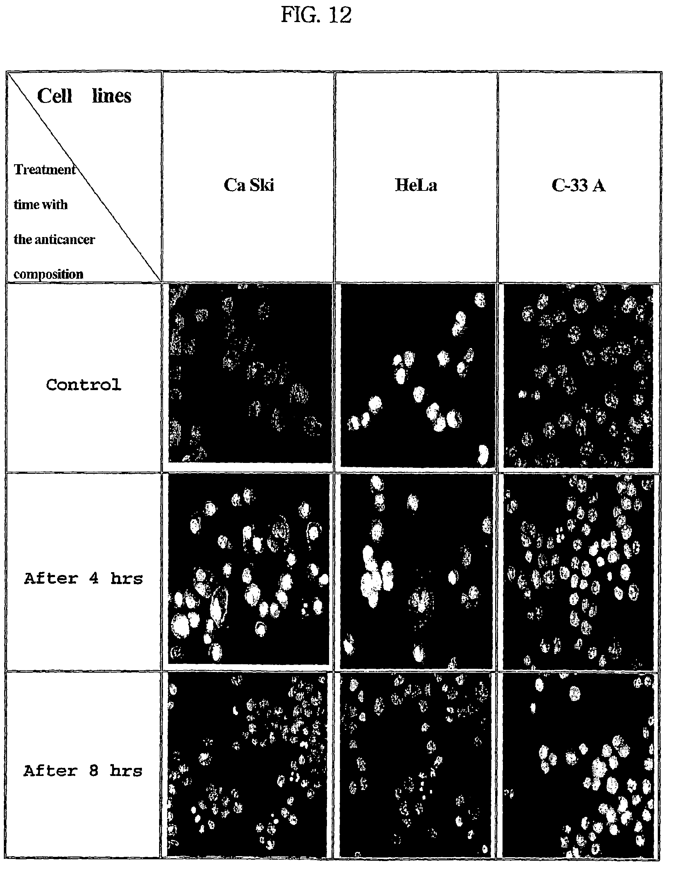

Sterilized cover glasses were placed into 60-mm culture dishes, Ca Ski, HeLa and C-33 A cells were plated at a density of 1×10⁴ cells, and cultured overnight. Thereafter, the cells were treated with 100 µl of the anticancer composition prepared in the Example 1 (the anticancer composition 3 of Table 1). After 24 hrs, the cells were washed with PBS twice, and fixed with 3.7% formaldehyde. After washing with PBS three times, the cells were stained with 4 µg/ml of a DAPI (4',6'-diamino-2-phenylinylindole) solution (Sigma, USA) at room temperature for 10 min. After washing with PBS three times and then distilled water twice, the stained cells fixed on the cover glasses were observed under a fluorescence microscope (Olympus Optical Co., Ltd., USA). The results are given in FIG. 12.

As shown in FIG. 12, in Ca Ski, HeLa and C-33 A cells not treated with the anticancer composition obtainable by the present invention, normal nuclei were observed. In contrast, when treated with the anticancer composition, the three-kinds cells showed morphological changes in nuclei by apoptosis, for example, chromatin condensation, nuclear fragmentation and nuclear shrinkage). In detail, the edge of the condensed chromatin mass was observed to be unperfected, and the condensed chromatin was scattered irregularly around the nucleus. These results indicate that the anticancer composition obtainable by the present invention induces apoptosis in a tumor cell-specific manner.

### EXAMPLE 12: Evaluation of expression of papillomavirus proteins in uterine cancer cells

In order to determine whether the antiviral composition obtainable by the present invention reduced expression of papillomavirus proteins in uterine cancer cell lines, expression of E6 and E7 proteins of HPV type 16 or 18 was analyzed.

Ca Ski and HeLa cells were plated onto 96-well plates at a density of 2×10⁶ cells, 100 µl of the anticancer composition prepared in Example 1 (the anticancer composition 3 of Table 1) was added to each well, and the plates were incubated for 6, 12, 24 and 48 hrs according to the same method as in Example 4. Cells were harvested individually after the incubation, and lysed with lysis buffer (10mM Tris, 1mM EDTA, 1mM DTT, 1mM PMSF, protease inhibitor). The resulting cell lysate was subjected to SDS-electrophoresis, and the separated protein was transferred to an ECL nitrocellulose membrane (Amersham Life Science, U.K.). The membrane was then blocked with a blocking solution (5% skim milk in TBST; 10mM Tris-HCl, pH 8.0. 150 mM NaCl, 0.1% Tween 20) at 4°C overnight.

Thereafter, E6 and E7 proteins of HPV type 16 or 18 were investigated by immunoblotting analysis, as follows. Primary antibodies (Santa Cruz Biotechnology, USA) to E6 of HPV type 16 and E7 of HPV type 18 were diluted to 1:500 in the blocking solution. The blocked membrane was reacted with the diluted primary antibodies at 4°C overnight, and then with a 1:5000 dilution of a secondary antibody (goat anti-rabbit IgG-HRP; Santa Cruz Biotechnology, USA) in the blocking solution at room temperature for 1 hr. After each treatment of primary and secondary antibodies, the membrane was washed three times for 15 min per each washing, and exposed to an ECL Hyperfilm (Amersham Life Science, U.K.). The results are given in FIG. 13.

As shown in FIG. 13, in Ca Ski and HeLa cells treated with the antiviral composition obtainable by the present invention, expression of the oncogenic protein E6 of HPV type 16 was gradually reduced with the passage of time. In addition, in Ca Ski cells treated with the antiviral composition obtainable by the present invention, expression of the oncogenic protein E7 of HPV type 18 was gradually reduced with the passage of time. These results indicate that the antiviral composition obtainable by the present invention inhibits expression of the oncogenic proteins E6 and E7 of HPV type 16 or 18 in uterine cancer cells.

### EXAMPLE 13: Therapeutic effects of the present composition versus condyloma acuminata caused by papillomavirus

1 g of the ointment (the transdermal formulation (2) of the Example 3) prepared in the Example 3 was applied twice everyday to the vulva of six female patients with condyloma acuminata. As a result, the genital warts were treated within 2 to 12 weeks according to the size of the lesions. The genital warts in the vulva of one of the subjects before and after ointment application are photographed in FIG. 14.

### EXAMPLE 14: Analysis of cytopathic effect caused by enterovirus and poliovirus

In order to demonstrate that the antiviral composition obtainable by the present invention inhibits viral proliferation, cytopathic effect was detected when cells infected with enterovirus or poliovirus were treated with the antiviral composition.

Vero E6 cells (African green monkey kidney cells) were grown in MEM medium containing 10% FBS in a 37°C incubator, and plated onto 48-well plates at a density of 1×10⁵ cells one day before experiments. The cultured cells were washed with PBS before infection with enterovirus and poliovirus. Enterovirus and poliovirus were titrated by plaque assay, and stored at -70°C until use. 6 pfu of enterovirus and poliovirus were individually incubated in 37°C incubator for one hour, washed with PBS, and added to the cell culture medium, followed by incubation for 24 hrs. The cells infected with the viruses were then treated with the antiviral composition obtainable by the present invention (the composition of the above Table 1), and evaluated for formation of cytopathic effect inhibition. As controls, the cells were treated with citric acid alone, zinc alone and the human seminal fluid. The results are given in FIGS. 15 and 16.

When the cells were treated with the antiviral composition obtainable by the present invention, formation of cytopathic effect was found to be inhibited in over 60%. On the other hand, in the controls treated with citric acid alone, zinc alone and the human seminal fluid, formation of cytopathic effect was inhibited in below 50%.

These results indicate that the antiviral composition obtainable by the present invention inhibits viral proliferation and finally induce death of cells infected with viruses.

## Claims

1. Use of an anticancer composition comprising therapeutically effective amounts of citric acid, zinc and L-arginine as active substances along with a pharmaceutically acceptable carrier in the manufacture of a medicament for treating cancer in a patient, whereby apoptosis of tumor cells is induced.

2. Use of an antiviral composition comprising therapeutically effective amounts of citric acid, zinc and L-arginine as active substances along with a pharmaceutically acceptable carrier in the manufacture of a medicament for treating cancer in a patient, whereby cancer is caused by a viral infection and a reduction of expression of viral proteins is induced.

## Patentansprüche

1. Verwendung einer Antikrebs-Zusammensetzung, umfassend therapeutisch wirksame Mengen von Zitronensäure, Zink und L-Arginin als aktive Substanzen zusammen mit einem pharmazeutisch akzeptablen Träger zum Herstellen eines Medikaments zur Behandlung eines Krebspatienten, wobei Apoptose der Tumorzellen induziert wird.

2. Verwendung einer antiviralen Zusammensetzung, umfassend therapeutisch wirksame Mengen an Zitronensäure, Zink und L-Arginin als aktive Substanzen zusammen mit einem pharmazeutisch akzeptablen Träger, zum Herstellen eines Medikaments zur Behandlung von Krebs in einem Patienten, wobei der Krebs ausgelöst ist durch eine virale Infektion und eine Reduktion der Expression viraler Proteine induziert wird.

## Revendications

1. Utilisation d'une composition anticancéreuse comprenant des quantités thérapeutiquement efficaces d'acide citrique, de zinc et de L-arginine comme substances actives ainsi qu'un vecteur pharmaceutique-ment acceptable dans la fabrication d'un médicament pour traiter le cancer chez un patient, où l'apoptose des cellules tumorales est induite.

2. Utilisation d'une composition antivirale comprenant des quantités thérapeutiquement efficaces d'acide citrique, de zinc et de L-arginine comme substances actives ainsi qu'un vecteur pharmaceutiquement acceptable dans la fabrication d'un médicament pour traiter le cancer chez un patient, où le cancer est causé par une infection virale et une réduction de l'expression des protéines virales est induite.
